# DEMANDE DE BREVET EUROPEEN

(11) **EP 4 817 538 A2**
(43) Date de publication de la demande: **07.10.2026**
(21) Numéro de dépôt: 26200605.9
(22) Date de dépôt: 25.09.2019
(51) Int. Cl.: A61B 17/34

(54) **DISPOSITIF POUR CHARGEMENT DE GREFFONS CAPILLAIRES DANS UN INSTRUMENT D'IMPLANTATION DE GREFFONS**

(30) Priorité: 27.09.2018 FR 1858891
(62) Numéro(s) de document de la (des) demande(s) initiale(s) en application de l'article 76 CBE: 22153104.9 / 4 023 177; 19795605.5 / 3 856 271
(71) Demandeur: Engraft Technologies, Inc., Marina Del Rey, CA 90292 (US)
(72) Inventeur: La désignation de l'inventeur n'a pas encore été déposée
(74) Mandataire: Kraeh, Christian

(57) **Abrégé**

L'invention concerne un dispositif (14, 14') pour chargement de greffons capillaires dans un instrument d'implantation du type Implanteur de Choï, comportant un élément comprenant :
- un premier orifice (18a) connecté à un canal (17) débouchant dans une chambre (21) d'évacuation de fluides ;
- un deuxième orifice (22) apte à communiquer avec la chambre (21) d'évacuation de fluides et à définir un chemin avec le canal (17), de sorte à permettre l'introduction de l'aiguille (5) d'un instrument d'implantation (1) dans ledit chemin par le deuxième orifice (22) ;
- des moyens de connexion (23) de la chambre (21) d'évacuation de fluides à une source de vide (27) ;
le premier orifice (18a) étant destiné à être mis en communication avec une première tubulure (20) destinée à véhiculer un greffon (12), de sorte que le greffon (12) soit apte à être inséré dans l'aiguille (5) de l'instrument d'implantation (1).

## Description

La présente invention concerne un dispositif pour chargement de greffons capillaires au sein d'un instrument d'implantation desdits greffons dans la peau, et un procédé de transfert et de chargement de greffons capillaires au sein d'un instrument d'implantation desdits greffons dans la peau.

La présente invention trouve une application particulièrement importante, bien que non exclusive, dans le domaine du traitement chirurgical de la calvitie par greffes de cheveux.

Une technique connue de greffe de cheveux consiste à prélever des fragments cutanés, généralement de forme cylindrique et de très faible diamètre, appelés greffons ou micro-greffons, comprenant généralement une à trois racines de cheveux nommées unités folliculaires, à l'arrière du cuir chevelu, à les charger dans un instrument d'implantation et à les réimplanter au niveau des zones chauves.

Pour le prélèvement de greffons, on connaît des appareils de découpe de greffons comprenant, un moyen de découpe cutanée comprenant un emporte-pièce de type à aiguille creuse à extrémité libre circulaire et tranchante monté sur un manche. Ce type d'emporte-pièce est nommé micro punch. Il peut soit être manuel, soit comporter un moteur électrique rotatif actionné par un opérateur.

Le procédé de prélèvement de greffons à l'aide de ce type d'appareil de découpe consiste à disposer le micro punch en regard du greffon à prélever, où les cheveux ont été préalablement coupés à environ un millimètre de longueur, puis à enfoncer le micro punch dans le cuir chevelu par un mouvement de rotation, sur quelques millimètres de profondeur en suivant l'axe d'émergence du ou des cheveux du greffon. Le micro punch est ensuite retiré du cuir chevelu, et le greffon prédécoupé est extrait du cuir chevelu par traction à l'aide de pinces très fines, avant d'être placé dans un réservoir comportant une solution de conservation. Le greffon est alors apte à être chargé dans un instrument d'implantation afin d'être réimplanté au niveau des zones dégarnies ou chauves.

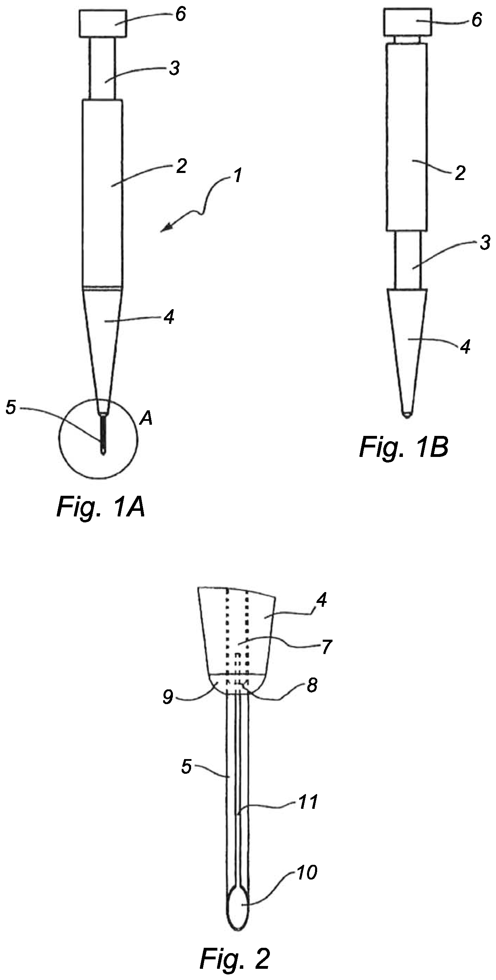

Le chargement d'un greffon dans un instrument d'implantation du type Implanteur de Choï, tel que décrit au regard des figures 1A, 1B et 2, est usuellement effectué manuellement. Le greffon est saisi manuellement entre les mors d'une pince fine maintenue par un opérateur, puis inséré dans l'aiguille creuse dudit instrument, par une fente longitudinale présente dans cette aiguille.

On peut comprendre aisément que l'étape de chargement manuelle du greffon dans l'aiguille d'implantation est une manœuvre longue et très délicate nécessitant une extrême précision rallongeant le temps d'intervention. En effet, l'aiguille d'un instrument d'implantation est généralement réalisée en acier inoxydable, et présente des dimensions très petites, de l'ordre de 10 millimètres de longueur sur sa partie libre dépassant de l'instrument d'implantation, d'un diamètre extérieur de l'ordre de 0,7 à 1 millimètre, et d'épaisseur de paroi très fine de l'ordre de 0,1 millimètre. Il est donc nécessaire pour un opérateur de bien visualiser et positionner la fente afin d'y insérer manuellement très délicatement le greffon.

En outre, une partie même minime du greffon peut se retrouver à l'extérieur de l'aiguille, au travers de la fente, et peut alors être abîmée ou sectionnée par étranglement dans la fente de par son étroitesse lors de son positionnement à l'intérieur de l'aiguille. Ceci a pour conséquence de rendre le greffon impropre à son implantation. Un changement de greffon est alors nécessaire.

Dans certains cas le prélèvement des greffons peut être effectué à l'aide d'un système de prélèvement par aspiration, comportant un appareil de découpe tel que décrit précédemment. Ce système de prélèvement par aspiration comporte une tubulure connectée de manière étanche à l'appareil de découpe et raccordé à un réservoir comportant une solution de conservation, et des moyens de mise au vide de la tubulure.

L'implantation des greffons au niveau des zones chauves se fait habituellement de deux manières différentes : l'implantation indirecte et l'implantation directe.

Dans l'implantation indirecte, les greffons sont insérés un par un dans des micro-incisions cutanées préalablement réalisées au niveau de la peau chauve. L'insertion des greffons se fait habituellement manuellement à l'aide de micro-pinces.

Dans l'implantation directe, les greffons sont directement injectés dans la peau chauve, à l'aide d'un instrument d'implantation, sans nécessité de réaliser des micro-incisions préalables. L'instrument d'implantation le plus communément employé, tel que décrit dans la demande US 6,461,369 dénommé « Implanteur de Choï » est constitué d'un corps prolongé par une aiguille creuse rétractable cylindrique à extrémité libre biseautée et tranchante au sein de laquelle est disposé le greffon à implanter dans le cuir chevelu. Une tige coulissante au sein de l'aiguille permet de libérer et maintenir le greffon dans le cuir chevelu lors du retrait de l'aiguille au sein de l'instrument d'implantation. L'aiguille est remarquable en ce qu'elle présente une ouverture étroite longitudinale ou fente permettant d'y charger manuellement le greffon à retro saisi à l'aide d'une pince très fine.

Un tel instrument d'implantation présente l'avantage de s'affranchir de la réalisation préalable de nombreuses micro-incisions cutanées au niveau des zones chauves mais présente malheureusement plusieurs inconvénients. En effet, le chargement manuel du greffon dans l'aiguille est long, très délicat, fastidieux et traumatisant pour le greffon dont la tête préalablement saisie par une pince se trouve étranglée par les bords de la fente étroite de l'aiguille. Le greffon peut se retrouver ainsi amputé de sa tête et donc impropre à son implantation. D'autre part, il est souvent nécessaire de disposer d'un personnel qualifié d'au moins deux assistants opératoires dont l'unique tâche consiste à placer sur une table les greffons dans plusieurs instruments d'implantation qui sont échangés successivement manuellement avec l'opérateur principal chargé de l'implantation proprement dite des greffons dans le cuir chevelu. Il est donc nécessaire de disposer d'un nombre important d'instruments à charger afin d'optimiser le temps opératoire d'implantation. Ces manipulations longues et délicates augmentent également le risque de blessure accidentelle des opérateurs lors de l'échange des instruments.

Une solution connue consiste à prélever et charger les greffons dans un instrument d'implantation en une seule étape. Cette solution est décrite dans le brevet FR 2 696 334 qui présente un appareil de découpe de greffons relié à un instrument d'implantation par une tubulure.

Une autre solution décrite dans le brevet US 7 452 367 consiste à charger des greffons disposés par exemple dans un réservoir contenant une solution de conservation, dans un instrument d'implantation présentant un orifice relié à une tubulure sous vide. Ainsi, le chargement des greffons dans l'instrument d'implantation permet de s'affranchir d'un chargement manuel dans l'aiguille de l'instrument d'implantation.

Cependant ces solutions ne sont pas compatibles avec des instruments d'implantation du type « Implanteur de Choï » existants. En effet, les instruments d'implantation tels que décrits dans les brevets FR 2 696 334 et US 7 452 367 présentent, contrairement aux Implanteurs de Choï, des orifices permettant leur liaison à des canaux ou tubulures permettant entre autre l'aspiration d'un greffon par aspiration sous vide

La présente invention a pour but de remédier aux inconvénients énumérés ci-dessus en proposant un dispositif pour chargement de greffons capillaires dans un instrument d'implantation du type Implanteur de Choï, permettant de charger simplement, rapidement, sans traumatisme et en toute sécurité des greffons au sein de l'instrument d'implantation, pour un coût de main d'œuvre réduit, sans nécessiter de modifier la structure de l'instrument d'implantation, et un procédé de chargement d'un instrument d'implantation du type Implanteur de Choï grâce à un tel dispositif pour chargement de greffons.

Le but de la présente invention vise à éliminer les manipulations délicates et les inconvénients énumérés ci-dessus en proposant un dispositif plus adapté à la pratique en permettant de charger rapidement et de manière aisée un greffon dans l'aiguille d'un instrument d'implantation du type Implanteur de Choï, sans traumatisme et en toute sécurité.

Dans ce but l'invention propose un dispositif pour chargement de greffons capillaires dans un instrument d'implantation du type Implanteur de Choï, caractérisé en ce qu'il comporte un élément comprenant :
- un premier orifice connecté à un canal débouchant dans une chambre d'évacuation de fluides ;
- un deuxième orifice apte à communiquer avec la chambre d'évacuation de fluides et à définir un chemin avec le canal, de sorte à permettre l'introduction de l'aiguille d'un instrument d'implantation dans ledit chemin par le deuxième orifice ;
- des moyens de connexion de la chambre d'évacuation de fluides à une source de vide ;
le premier orifice étant destiné à être mis en communication avec une première tubulure destinée à véhiculer un greffon, de sorte que le greffon soit apte à être inséré dans l'aiguille de l'instrument d'implantation.

Ainsi, le dispositif pour chargement de greffon selon l'invention présente de nombreux avantages par rapport à l'art antérieur. Il permet un gain de temps considérable lors de sa manipulation et il n'est plus nécessaire de disposer d'un nombre important d'instruments d'implantation préalablement chargés manuellement. Un seul instrument d'implantation peut suffire à l'accomplissement de l'intervention, ce qui représente un gain économique conséquent.

En outre, il n'est plus nécessaire de se soucier du bon positionnement de la fente de l'aiguille de l'instrument d'implantation lors du chargement du greffon dans l'aiguille dudit instrument, compte-tenu du fait qu'une fois cette dernière introduite dans le dispositif pour chargement de greffon selon l'invention, la portion libre de la fente communique latéralement librement autour de son axe sur 360 ° avec la chambre d'évacuation de fluides. Le chargement est assuré lors de la mise sous vide de la chambre d'évacuation de fluides.

De plus, le chargement d'un instrument d'implantation grâce au dispositif selon l'invention peut être effectué par un seul opérateur utilisant le même instrument, ce qui a pour effet d'empêcher tout risque de piqûre accidentelle grâce à l'absence d'échange physique d'instruments entre différents opérateurs.

Selon d'autres caractéristiques de l'invention, le dispositif de l'invention comporte l'une ou plusieurs des caractéristiques optionnelles suivantes considérées seules ou selon toutes les combinaisons possibles.

Selon une caractéristique, la chambre d'évacuation de fluides présente des dimensions telles qu'elle soit apte à permettre à la fois un passage de fluide tel que l'air ou l'eau entre le canal et les moyens de connexion de la chambre d'évacuation de fluides à une source de vide, et le chargement d'un greffon dans l'aiguille d'un instrument d'implantation du type Implanteur de Choï introduite dans le canal via le deuxième orifice.

Selon une caractéristique, le canal est rectiligne.

Selon une autre caractéristique, les moyens de connexion de la chambre d'évacuation de fluides à une source de vide sont un conduit de mise sous vide disposé dans le dispositif pour chargement de greffon, sensiblement parallèlement au canal, destiné à être connecté à la source de vide par un orifice de sortie.

En variante, les moyens de connexion de la chambre d'évacuation de fluides à une source de vide sont un conduit de mise sous vide disposé latéralement et perpendiculairement au canal.

Selon un mode de réalisation, le dispositif pour chargement de greffons présente une forme sensiblement cylindrique avec une face proximale, une face distale et une face longitudinale reliant les faces proximale et distale.

Selon ce mode de réalisation, la face proximale comporte le deuxième orifice et la face distale comporte le premier orifice du canal.

Toujours selon ce mode de réalisation, dans la variante selon laquelle les moyens de connexion de la chambre d'évacuation de fluides à une source de vide sont un conduit de mise sous vide disposé dans le dispositif pour chargement de greffon, sensiblement parallèlement au canal, destiné à être connecté à la source de vide par un orifice de sortie, l'orifice de sortie du conduit de mise sous vide est disposé sur la face distale du dispositif pour chargement de greffons.

En variante, l'orifice de sortie du conduit de mise sous vide est disposé latéralement, sur la face longitudinale du dispositif de chargement de greffons.

Le premier orifice du canal est avantageusement disposé au centre de la face distale du dispositif pour chargement de greffon.

Selon cette caractéristique, le deuxième orifice est disposé au centre de la face proximale du dispositif pour chargement de greffon.

Ainsi, le premier orifice et le deuxième orifice sont alignés dans l'axe du canal, ou coaxiaux.

Le dispositif pour chargement de greffon comporte de préférence des moyens de connexion à des tubulures permettant de relier à distance respectivement le canal à un réservoir de greffons, et le conduit à une source de vide.

L'invention concerne en outre un procédé d'utilisation du dispositif tel que décrit précédemment pour le chargement d'un greffon capillaire dans un instrument d'implantation du type Implanteur de Choï, comportant les étapes suivantes :
- on connecte une première tubulure au premier orifice, via un moyen de connexion,
- on introduit l'aiguille d'un instrument d'implantation, de manière étanche, dans le deuxième orifice, de sorte à ce qu'elle pénètre dans au moins une partie du canal,
- on approche l'extrémité libre de la première tubulure au contact d'un greffon disposé au sein d'une solution dans un réservoir ou récipient, et
- on met la chambre d'évacuation de fluides sous vide.

L'invention concerne également un plateau de stockage de greffons configuré pour faciliter l'aspiration d'un greffon dans un dispositif pour le chargement de greffon tel que décrit précédemment, le plateau de stockage comportant au moins un puit configuré pour contenir un greffon et dont les dimensions permettent de maintenir le greffon dans une position donnée adaptée pour permettre l'aspiration du greffon dans un dispositif pour le chargement de greffon tel que décrit précédemment.

Ainsi, lorsqu'un greffon est introduit dans un puit avec sa partie épidermique proche de l'ouverture du puit, le greffon est maintenu dans cette position, de sorte que le greffon puisse être aisément aspiré dans le dispositif pour le chargement de greffon.

Selon une caractéristique, le puit présente un diamètre de l'ordre de 1,5 mm et une profondeur de l'ordre de 8 mm.

Selon une caractéristique, le plateau de stockage comporte une pluralité de puits alignés sur plusieurs rangées, équidistants et espacés entre eux d'environ 5 mm.

L'invention concerne également un dispositif d'aspiration de greffons configuré pour aspirer un greffon dans un dispositif pour chargement de greffon tel que décrit précédemment.

Le dispositif d'aspiration de greffons est un robot comportant un support et un logement pour un tube rigide configuré pour être relié à la première tubulure d'un dispositif pour chargement de greffon tel que décrit précédemment, le logement étant mobile par rapport au support et configuré pour être déplacé afin de positionner le tube rigide au contact d'un greffon.

Selon une caractéristique, le logement est mobile selon trois axes orthogonaux, relativement au plateau de stockage de greffons.

Selon une caractéristique, le dispositif d'aspiration comporte en outre :
- une caméra configurée pour enregistrer une image d'un greffon disposé dans un récipient, et
- un contrôleur configuré pour analyser la position précise du greffon.

L'invention concerne un ensemble comprenant un dispositif pour chargement de greffon et un dispositif d'aspiration de greffon tels que décrits précédemment.

L'invention concerne un ensemble comprenant un dispositif pour chargement de greffon, un dispositif d'aspiration de greffon et un plateau de stockage tels que décrits précédemment.

L'invention sera mieux comprise à la lecture de modes de réalisation donnés ci-après à titre d'exemple non limitatifs.

La description se réfère aux dessins qui l'accompagnent dans lesquels :
- la figure 1A est une vue de face d'un instrument d'implantation de greffons capillaires dans le cuir chevelu d'un patient, communément employé dans l'art antérieur, avec son aiguille en position extériorisée ;
- la figure 1B est une vue de face de l'instrument d'implantation de greffons capillaires de la figure 1A avec son aiguille en position rétractée ;
- la figure 2 est une vue agrandie du détail de la zone A de la figure 1A ;
- les figures 3 à 3C sont des vues en perspective des différentes étapes de l'art antérieur de chargement manuel d'un greffon dans l'aiguille d'un instrument d'implantation selon la figure 1A ;
- les figures 4A et 4B sont des vues en perspective du dispositif pour chargement de greffons capillaires selon la présente invention ;
- la figure 5 est une vue en coupe du dispositif de la figure 4A, selon le plan P désigné sur la figure 4A ;
- la figure 6 est une vue en perspective illustrant un mode de réalisation du procédé de chargement d'un greffon dans un instrument d'implantation selon les figures 1A,1B et 2, grâce au dispositif selon l'invention ;
- les figures 7A à 7E sont des vues en coupe illustrant les différentes étapes successives du chargement d'un greffon dans l'aiguille d'un instrument d'implantation selon la figure 1A grâce au dispositif selon l'invention ;
- Le figure 8 est une vue en coupe illustrant un autre mode de réalisation du dispositif pour chargement de greffon ;
- La figure 9 est une vue en perspective illustrant un mode d'utilisation du dispositif de la figure 8 ;
- La figure 10 est une vue en perspective d'un mode de réalisation d'un plateau de stockage de greffons selon l'invention ;
- Les figures 11 et 12 sont des vues en perspective de deux modes de réalisation d'un dispositif d'aspiration de greffons selon l'invention ;
- La figure 13 est une vue en perspective illustrant un mode d'utilisation du dispositif de la figure 11 ;
- La figure 14 est une vue en perspective illustrant un mode d'utilisation du dispositif de la figure 12.

Dans la description qui va suivre et dans les revendications, des composants identiques, similaires ou analogues seront désignés par les mêmes chiffres de référence et on emploiera les termes « avant », « arrière », « horizontal », « vertical », « supérieur », « inférieur », etc. à titre non limitatif et en référence aux dessins afin de faciliter la description.

Sur les figures 1A, 1B et 2 on voit que l'instrument 1 d'implantation de greffons capillaires comporte un corps allongé présentant un manchon 2 de forme cylindrique au sein duquel coulisse un piston 3 prolongé à une de ses extrémités par une partie 4 effilée de forme conique, et à son extrémité opposée par un bouton poussoir 6.

Le manchon 2 est disposé entre la partie 4 effilée de forme conique et le bouton poussoir 6, et présente une longueur inférieure à la distante entre la partie 4 effilée de forme conique et le bouton poussoir 6, de sorte à permettre le coulissement du piston 3 par rapport au manchon 2.

La partie 4 effilée de forme conique présente une base de largeur sensiblement identique à celle du manchon, et une pointe 9 arrondie.

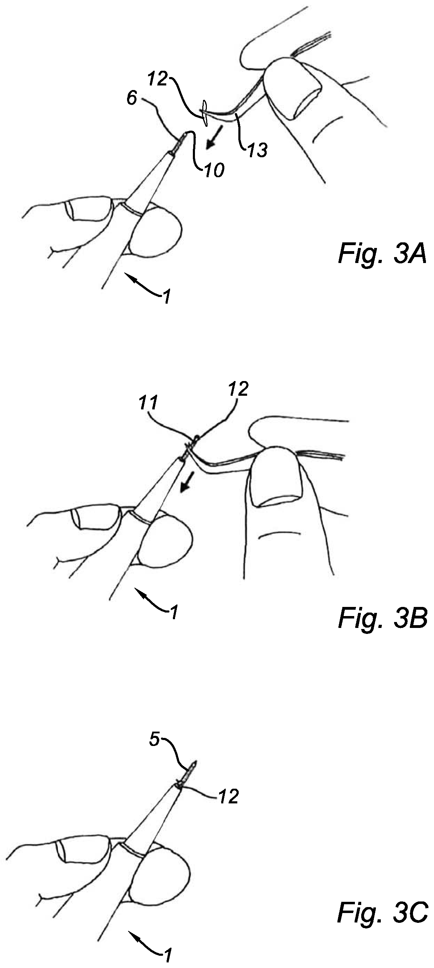

Une aiguille 5 d'implantation creuse, de forme cylindrique, propre à contenir un greffon capillaire 12 (figures 3A à 3C), est fixée solidairement au manchon 2 par des moyens d'accouplement (non représentés). L'aiguille 5 est mobile entre une position rétractée (figure 1B) à l'intérieur de la partie 4 effilée de forme conique du piston 3 lorsque ce dernier est mobilisé par rapport au manchon 2 par le biais du bouton poussoir 6, et une position déployée (figure 1A) dans laquelle elle est en saillie de la partie 4 effilée du piston 3. La longueur L de l'aiguille 5 d'implantation en saillie est de l'ordre de 8 à 10 mm.

Une tige 7 coulissante de forme cylindrique est disposée à l'intérieur de l'aiguille 5 creuse.

La tige 7 coulissante, de diamètre sensiblement inférieur au diamètre intérieur de l'aiguille 5, est fixée solidairement au piston 3 par son extrémité proximale, proche du bouton poussoir 6, par des moyens d'accouplement (non représentés), et présente une extrémité distale 8 opposée, libre disposée à hauteur de la pointe 9 de la partie 4 effilée de forme conique, de telle sorte qu'elle puisse servir de butée et libérer un greffon (non représenté) hors de l'aiguille 5 lors du retrait de cette dernière à l'intérieur du piston3.

L'aiguille creuse 5 présente une extrémité libre 10 biseautée et tranchante propre à pénétrer la peau ainsi que, sur une partie de sa longueur, une ouverture longitudinale étroite ou fente 11 permettant d'y insérer un greffon 12 généralement par enfilement ou traction à l'aide d'une pince 13 comme illustré sur les figures 3A à 3C.

Sur la figure 3A on voit que le greffon 12 est saisi manuellement à sa partie supérieure entre les mors d'une pince 13 fine maintenue par un opérateur. Le greffon 12 est approché au regard de l'extrémité libre 10 de l'aiguille d'implantation creuse 5 de l'instrument 1 d'implantation. Le greffon 12 est alors inséré très délicatement par traction ou enfilement à l'intérieur de l'aiguille 5 creuse à travers la fente 11 de l'aiguille 5 jusqu'à ce que le greffon y soit totalement chargé et logé comme illustré sur les figures 3B et 3C. L'instrument 1 d'implantation est alors prêt à être utilisé pour l'implantation du greffon 12 dans la peau.

On peut comprendre aisément que l'étape de chargement manuelle du greffon dans l'aiguille d'implantation est une manœuvre longue et très délicate nécessitant une extrême précision rallongeant le temps d'intervention. En effet, l'aiguille 5, généralement réalisée en acier inoxydable, présente des dimensions très petites, de l'ordre de 10 millimètres de longueur sur sa partie libre, d'un diamètre extérieur de l'ordre de 0,7 à 1 millimètre, et d'épaisseur de paroi très fine de l'ordre de 0,1 mm. L'aiguille est ouverte sur toute sa longueur libre par la fente 11 dont la largeur est de l'ordre de 0,2 millimètre sur environ 10 millimètres de longueur à partir du bord postérieur de l'extrémité 10 biseautée de l'aiguille 5. Il est donc nécessaire pour un opérateur de bien visualiser et positionner la fente 11 afin d'y insérer manuellement très délicatement le greffon saisi par la pince 13.

On comprend aisément qu'une partie même minime du greffon 12 puisse se retrouver à l'extérieur de l'aiguille 5 au travers de la fente 11 et puisse être sectionnée par étranglement dans la fente 11 de par son étroitesse lors de son positionnement à l'intérieur de l'aiguille 5, ce qui rend le greffon impropre à son implantation.

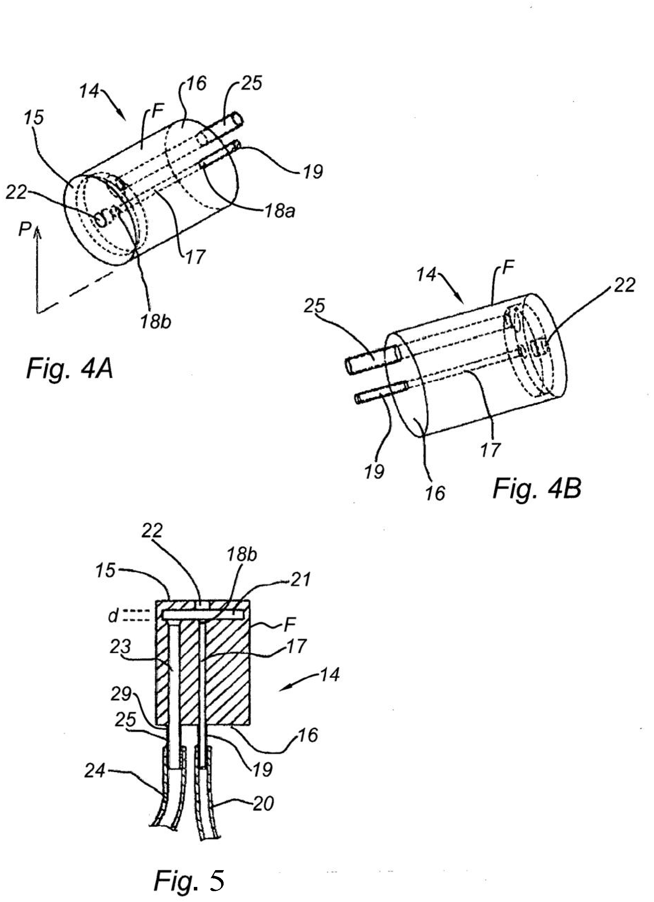

Les figures 4A, 4B et 5 sont des vues respectivement en perspective et en coupe d'un dispositif 14 pour chargement de greffons capillaires selon un mode de réalisation de la présente invention.

Le dispositif 14 pour chargement de greffons présente une forme cylindrique, une longueur d'environ 20 millimètres et un diamètre d'environ 16 millimètres. Il présente une face proximale 15 plane, une face distale 16 plane et une face longitudinale F reliant les faces proximale 15 et distale 16.

Le dispositif 14 pour chargement de greffons comporte une chambre 21 de forme circulaire aménagée à proximité de la face proximale 15. Il comporte en outre un canal central 17 longitudinal rectiligne de forme cylindrique qui présente un premier orifice 18a débouchant de la face distale 16, et une ouverture 18b opposée au premier orifice 18a, débouchant dans la chambre 21.

La face proximale 15 comporte un deuxième orifice 22 qui débouche dans la chambre 21. Le deuxième orifice 22 est apte à recevoir, de manière étanche, l'aiguille 5 (non représentée) d'un instrument d'implantation tel que décrit précédemment, ladite aiguille étant apte à traverser la chambre 21 pour rejoindre le canal central 17.

Ainsi, le canal central 17 est apte à recevoir axialement sur une partie de sa longueur l'aiguille d'un instrument d'implantation tel que décrit au regard des figures 1A, 1B et 2, introduite à travers l'orifice central 22.

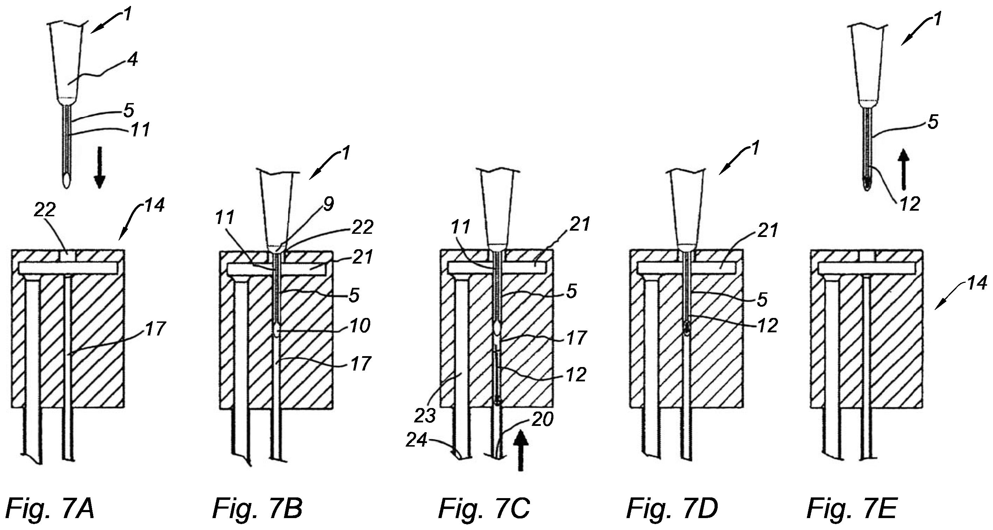

Le canal central 17 présente en saillie vers l'extérieur, au niveau de son premier orifice 18a, un moyen de connexion étanche 19 à une première tubulure 20 (figure 5) souple de longueur variable et d'un diamètre intérieur d'environ 1 à 1,2 mm propre à contenir et véhiculer un greffon 12 (figures 7C à 7E).

La chambre 21 est en communication à une source de vide située à distance par l'intermédiaire d'un conduit de mise sous vide 23 comportant un orifice de sortie 29 débouchant sur la face distale 16 et relié à une deuxième tubulure 24 (figure 5) par un moyen de connexion 25.

La chambre 21 présente une hauteur « d » d'environ 1,5 millimètre, suffisamment importante pour permettre un passage aisé de fluides (air ou eau) entre le canal central 17 et le canal latéral 23, et suffisamment étroite pour permettre le chargement complet d'un greffon dans l'aiguille 5 d'un instrument d'implantation tel que décrit précédemment au regard des figures 1 à 2, lors de son introduction dans le canal central 17 via le deuxième orifice 22, comme il sera décrit en détail au regard des figures 7A à 7E.

Le deuxième orifice 22 du dispositif 14 pour chargement de greffons présente un diamètre suffisamment large pour permettre l'introduction aisée de l'aiguille 5 de l'instrument d'implantation et suffisamment étroit pour réaliser une butée et un joint étanche avec la pointe 9 de l'instrument d'implantation tel que décrit précédemment.

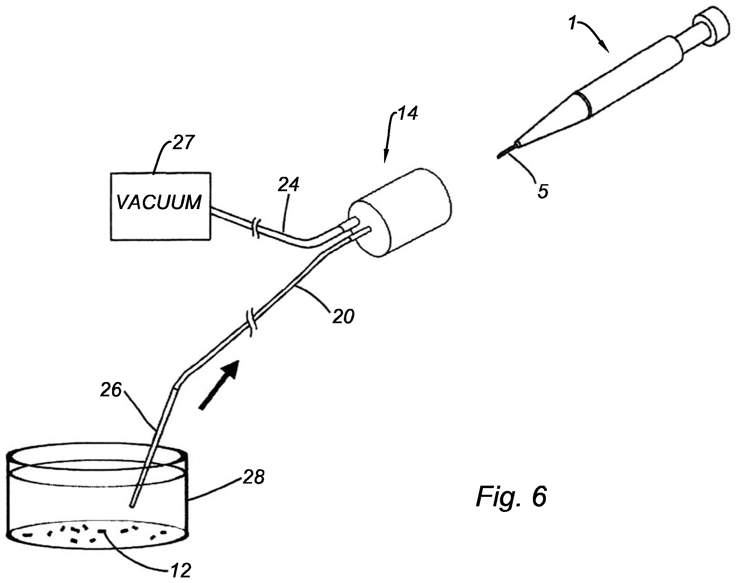

La figure 6 illustre en perspective un mode d'utilisation du dispositif 14 pour chargement de greffons de la présente invention.

Le dispositif 14 pour chargement de greffons, pouvant être fixé par exemple sur une bague d'un doigt d'un opérateur (non représentée), est relié par la première tubulure 20 à un tube rigide 26 par exemple en acier inoxydable, de diamètre intérieur d'environ 1 à 1,2 mm suffisant pour permettre le passage d'un greffon 12.

L'extrémité libre du tube 26 est destinée à être mise au contact de la partie épidermique d'un greffon 12 baignant parmi d'autres greffons au fond d'un récipient ou réservoir à greffons 28 préalablement rempli de sérum physiologique ou autre liquide de conservation, en vue de son aspiration dans le dispositif 14 pour chargement de greffons.

Le dispositif 14 pour chargement de greffons est également relié par la deuxième tubulure 24 à une source de vide 27 pouvant être actionnée par un opérateur. L'instrument 1 d'implantation décrit à la figure 1 est positionné axialement en face du dispositif 14 pour chargement de greffons, prêt à être chargé par un greffon 12 comme décrit ci-après.

Dans ce mode d'utilisation, l'aspiration d'un greffon 12 dans le dispositif 14 pour chargement de greffons, est réalisée manuellement par un opérateur qui manipule le tube 26 afin de le mettre en contact avec la partie épidermique d'un greffon 12.

Les figures 7A à 7E sont des vues en coupe illustrant les différentes étapes du chargement d'un greffon 12 dans l'aiguille 5 d'un instrument 1 d'implantation grâce au dispositif 14 pour chargement de greffons selon l'invention.

Sur les figures 7A et 7B, on voit que l'aiguille 5 de l'instrument d'implantation 1 est aligné dans l'axe du canal central 17 au regard du deuxième orifice 22 du dispositif 14 pour chargement de greffons. L'aiguille d'implantation 5 est alors introduite à frottement dans le canal central 17 jusqu'à ce que la pointe 9 de l'instrument d'implantation 1 vienne buter contre le bord circulaire du deuxième orifice 22 en y formant un joint circulaire étanche aux fluides.

En position d'accouplement entre l'instrument d'implantation 1 et le dispositif 14 pour chargement de greffons, l'aiguille d'implantation 5 forme un espace cylindrique ouvert à son extrémité libre 10 dans le prolongement du canal central 17 et ouvert latéralement dans la chambre 21 par la portion libre de la fente 11.

En référence aux figures 7C et 7D, la mise en dépression par l'intermédiaire de la source de vide 27 commandée par un opérateur, de la chambre 21 via le conduit 23 et la deuxième tubulure 24, provoque une dépression successivement dans la chambre 21, dans l'aiguille 5 via la portion libre de la fente 11, dans le canal central 17, dans la première tubulure 20 et dans le tube 26 (figure 6) située au regard de la partie épidermique d'un greffon 12, provoquant son aspiration et son cheminement en une fraction de seconde jusque dans le canal central 17 pour s'introduire et se bloquer à l'intérieur de l'aiguille 5 au regard de la chambre 21, compte-tenu du fait que la largeur de la fente 11 d'environ 0,2 mm est suffisamment large pour laisser passer des fluides mais suffisamment étroite pour bloquer et immobiliser le greffon dont le diamètre est plus large d'environ 0,9 à 1 millimètres.

Une fois le greffon 12 chargé et positionné à l'intérieur de l'aiguille 5 de l'instrument d'implantation 1, ce dernier est alors séparé par traction du dispositif 14 pour chargement de greffons, comme illustré sur la figure 7E, en vue de son utilisation finale pour l'implantation du greffon dans la peau ou le cuir chevelu d'un patient.

L'instrument 1 d'implantation libéré du greffon 12 est alors à nouveau introduit dans le dispositif 14 pour chargement de greffons afin d'y charger un deuxième greffon et ainsi de suite.

Il est également envisageable de disposer de plusieurs dispositifs 14 dont le diamètre du canal central 17 varie pour s'adapter au diamètre extérieur de l'aiguille de l'instrument d'implantation à charger, par exemple 0,8 ou 0,9 ou 1 millimètre de diamètre, en fonction de la taille des greffons. De tels dispositifs sont rapidement interchangeables par l'opérateur en cours d'intervention.

Le dispositif 14 pour chargement de greffons selon l'invention peut être aisément fabriqué en matière plastique rigide transparente, par injection dans un moule ou par usinage et collage, telle qu'en polycarbonate ou méthacrylate de méthyle.

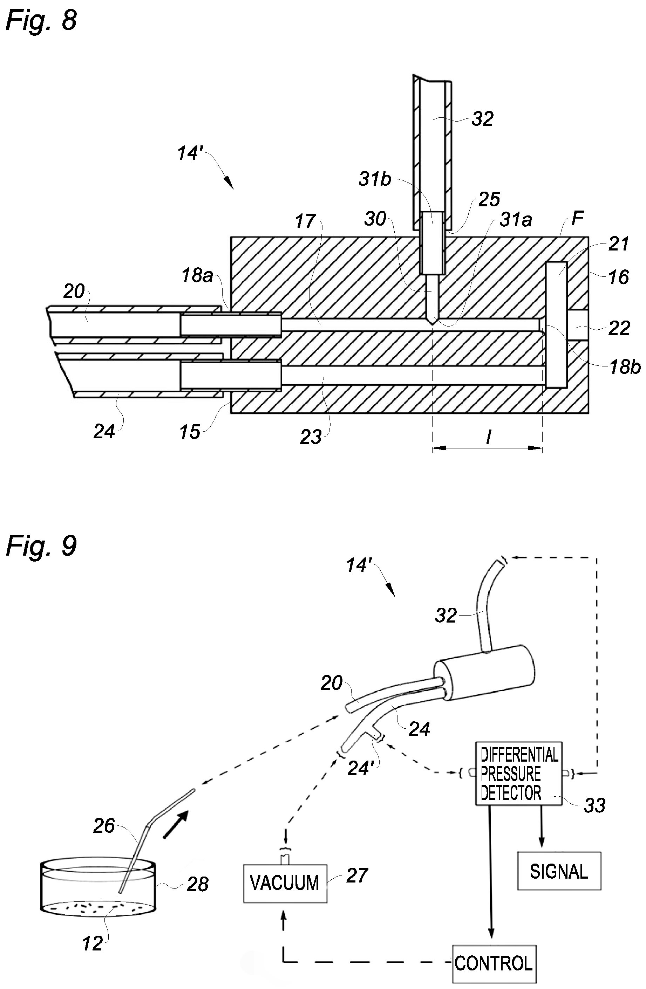

La figure 8 est une vue en coupe d'un dispositif 14' pour chargement de greffons capillaires selon un autre mode de réalisation.

A la différence du dispositif 14 pour chargement de greffons selon le mode de réalisation des figures 4A, 4B et 5, le dispositif 14' pour chargement de greffons de la figure 8 présente un canal accessoire 30 débouchant perpendiculairement depuis la face longitudinale F du dispositif, dans le canal central 17.

Le canal accessoire 30 comporte un orifice accessoire 31a débouchant dans le canal central 17 et une ouverture accessoire 31b opposée à l'orifice accessoire 31a et disposé dans la face longitudinale F du dispositif.

L'orifice accessoire 31a est situé à une distance « l » de l'ouverture 18b du canal central 17 débouchant dans la chambre 21. La distance « l » est égale à la longueur L de l'aiguille 5 d'implantation d'un instrument d'implantation tel que décrit au regard des figures 1A, 1B et 2.

Le canal accessoire 30 présente un diamètre sensiblement identique au diamètre du canal central 17.

En outre, le canal accessoire 30 présente en saillie vers l'extérieur, au niveau de son ouverture accessoire 31b, un moyen de connexion étanche 25 à une troisième tubulure 32 configurée pour être reliée à un capteur de pression 33 (figure 9).

Comme le montre la figure 9, le capteur de pression 33 est également relié à la deuxième tubulure 24, qui permet de relier le dispositif 14' pour chargement de greffons à la source de vide 27. La liaison entre le capteur de pression 33 et la deuxième tubulure 24 est effectuée via une canule 24'.

Ainsi, le capteur de pression 33 est apte à détecter une variation brutale de pression entre l'orifice accessoire 31a et la source de vide 27. Une telle variation pourrait être provoquée par l'obstruction de l'aiguille 5 d'implantation par un greffon 12 aspiré dans le tube rigide 26 et la tubulure 20 selon les étapes de chargement d'un greffon décrites au regard des figures 7C et 7D.

Le capteur de pression 33 est assimilé à un contacteur pneumatique. Il permet d'indiquer à l'opérateur, par un signal sonore ou lumineux, la bonne présence du greffon dans l'aiguille 5 d'implantation.

Dans ce mode d'utilisation, l'aspiration d'un greffon 12 dans le dispositif 14' pour chargement de greffons, est réalisée manuellement par un opérateur qui manipule le tube 26 afin de le mettre en contact avec la partie épidermique d'un greffon 12 disposé dans un réservoir 28.

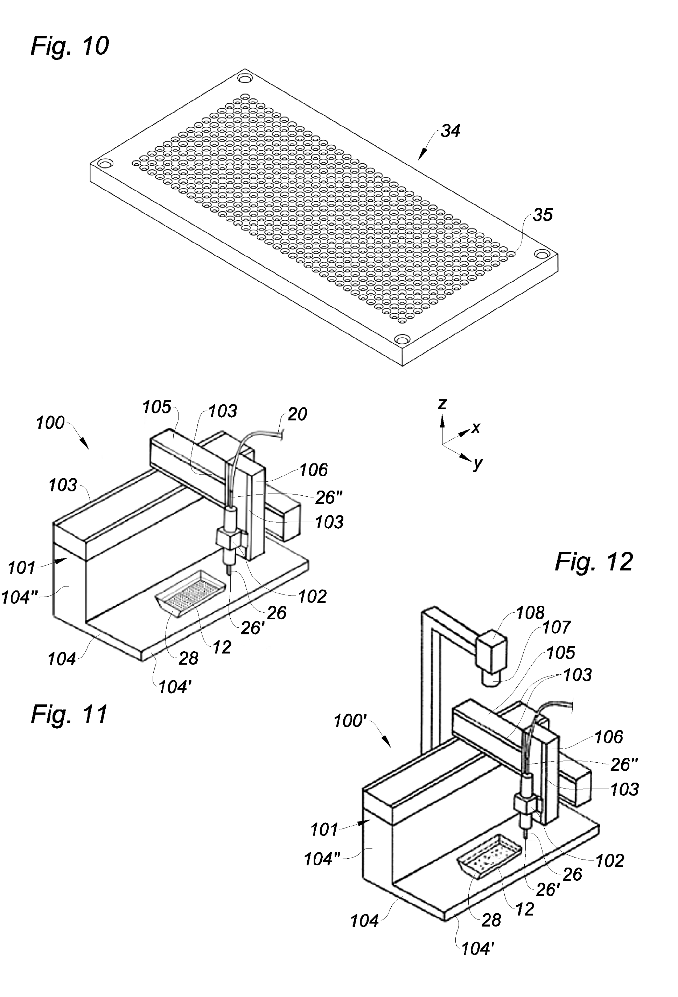

La figure 10 illustre un plateau 34 de stockage de greffons selon l'invention.

Le plateau 34 de stockage est rectangulaire, avec une longueur de l'ordre de 20 cm, une largeur de l'ordre de 10 cm et une hauteur de l'ordre de 1 cm. Il comporte quinze rangées de trente-cinq puits 35 configurés pour contenir chacun un greffon 12 (non représenté). Les puits 35 sont cylindriques, alignés, équidistants et espacés entre eux d'environ 5 mm

Chaque puit présente un diamètre de l'ordre de 1,5 mm et une profondeur de l'ordre de 8 mm. Ces dimensions permettent de maintenir le greffon dans une position donnée adaptée pour permettre l'aspiration du greffon dans un dispositif 14, 14' pour le chargement de greffon tel que décrit précédemment.

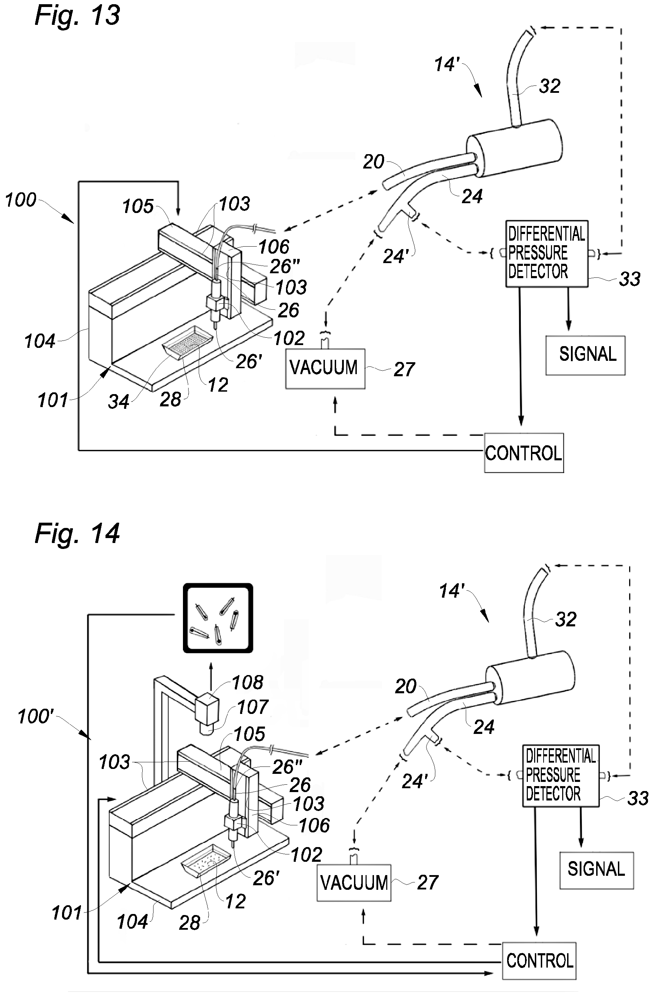

Le plateau de stockage 34 est configuré pour être placé dans un réservoir 28 (figure 13) légèrement plus grand, comportant du sérum physiologique.

La figure 11 illustre un mode de réalisation d'un dispositif d'aspiration 100 de greffons configuré pour aspirer un greffon 12 disposé dans un réservoir 28 à greffons, et pour l'insérer dans un dispositif 14, 14' pour chargement de greffon tel que décrit précédemment.

Le dispositif d'aspiration 100 de greffons est un robot comportant un support 101 pour un logement 102 pour un tube 26 rigide configuré pour être relié à la première tubulure 20 d'un dispositif 14, 14' pour chargement de greffon tel que décrit précédemment, le logement 102 étant configuré pour être déplacé selon trois axes orthogonaux, respectivement axe de la longueur x, axe de la profondeur y et axe de la hauteur z, grâce à des rails 103 disposés sur le support 101.

Le support 101 comprend une base 104 d'axe longitudinal correspondant à l'axe de la longueur x, en forme de L, comportant deux branches respectivement horizontale 104' et verticale 104''. La branche horizontale 104's'étend selon l'axe de la profondeur y et la branche verticale 104'' s'étend selon l'axe de la hauteur z.

Le support comporte en outre deux bras, respectivement horizontal 105 et vertical 106.

Le bras horizontal 105 s'étend à partir de la branche verticale 104'' de la base, parallèlement à la branche horizontale 104' et à l'aplomb de celle-ci. Il est configuré pour être déplacé selon l'axe de la longueur x, via des rails s'étendant selon l'axe de la longueur x sur la branche verticale 104'' de la base.

Le bras vertical 106 s'étend perpendiculairement au bras horizontal 105, selon l'axe de la hauteur z. Il est relié au bras horizontal 105 via des rails 103 s'étendant selon l'axe de la profondeur y sur le bras horizontal 105, de sorte à être mobile selon l'axe de la profondeur y.

Le logement 102 est relié au bras vertical 106 via des rails 103 s'étendant selon l'axe de la hauteur z sur le bras vertical 106, de sorte à être mobile selon l'axe de la hauteur z.

Le tube 26 s'étend selon l'axe de la hauteur z. Il présente une extrémité basse 26' libre et une extrémité haute 26'' configurée pour être relié à la première tubulure 20 d'un dispositif 14, 14' pour chargement de greffon tel que décrit précédemment, comme représenté sur la figure 13.

Le support permet de déplacer le logement 102 selon les axes orthogonaux de la longueur x, profondeur y et hauteur z, afin de positionner l'extrémité basse 26' du tube 26 au contact de la partie épidermique d'un greffon 12 disposé dans un réservoir 28 disposé sur la branche horizontale 104' de la base 104 du support 101.

Comme il sera vu au regard de la figure 13, l'aspiration d'un greffon 12 dans le dispositif 14 pour chargement de greffons, est réalisée mécaniquement par le dispositif d'aspiration 100 de greffons.

La figure 12 illustre un autre mode de réalisation d'un dispositif d'aspiration 100' de greffons

A la différence du mode de réalisation de la figure 11, le dispositif d'aspiration 100' de greffons, comporte une caméra 107 configurée pour enregistrer une image d'un greffon disposé dans un réservoir 28 disposé sur la branche horizontale 104' de la base 104 du support 101, et un contrôleur 108 configuré pour analyser la position précise du greffon 12.

Dans ce mode de réalisation, le déplacement du logement 102 permettant de déplacer le tube 26 au contact de la partie épidermique d'un greffon 12, est réalisé automatiquement grâce à la détection de la position d'un greffon 12 dans le réservoir 28.

La figure 13 illustre un mode d'utilisation du dispositif d'aspiration 100 d'un greffon de la figure 11, dans un dispositif 14' pour chargement de greffon de la figure 8.

A la différence du procédé tel que décrit au regard de la figure 9, l'aspiration d'un greffon 12 dans le dispositif 14' pour chargement de greffons, est réalisée mécaniquement par le dispositif d'aspiration 100 de greffons.

En outre, les greffons 12 sont disposés dans un plateau de stockage 34 tel que décrit au regard de la figure 10, afin de permettre une aspiration automatique des greffons. En effet, les greffons sont positionnés dans des puits 35 ( figure 10) du plateau de stockage 34, dans une position adaptée à leur aspiration dans le dispositif 14 pour chargement de greffon.

Ainsi, les déplacements du logement 102 sont prédéfinis selon la distance entre les puits.

Les déplacements du longement 102 sont commandés par le capteur de pression 33. Le capteur de pression permet ainsi de commander automatiquement le déplacement du tube 26 au regard d'un greffon selon un trajet préalablement programmé.

Dans la variante selon laquelle les greffons sont disposés dans un réservoir 28, sans plateau de stockage, le déplacement du logement 102 est réalisé manuellement par un opérateur afin de déplacer le tube 26 au contact de la partie épidermique d'un greffon 12 disposé dans le réservoir 28.

Dans une variante non représentée, le dispositif d'aspiration 100 est utilisé pour aspirer un greffon 12 dans un dispositif 14 pour chargement de greffon selon les figures 4A, 4B et 5.

Dans cette variante, l'aspiration d'un greffon est actionnée par un opérateur, grâce à une pédale (non représentée). Le déplacement du logement 102 est réalisé lors de l'arrêt de l'actionnement de la pédale par l'opérateur.

La figure 14 représente un mode d'utilisation du dispositif d'aspiration 100' d'un greffon de la figure 12, dans un dispositif 14' pour chargement de greffon de de la figure 8.

A la différence du procédé tel que décrit au regard de la figure 9, l'aspiration d'un greffon 12 dans le dispositif 14' pour chargement de greffons, est réalisée mécaniquement par le dispositif d'aspiration 100' de greffons.

En outre, l'aspiration est réalisée automatiquement grâce à la détection de la position des greffons par la caméra 107 et au contrôleur 108.

Dans une variante non représentée, le dispositif d'aspiration 100' est utilisé pour aspirer un greffon 12 dans un dispositif 14 pour chargement de greffon selon les figures 4A, 4B et 5.

Bien que le dispositif de chargement des greffons ait été décrit de forme cylindrique, il est envisageable qu'il soit de forme cubique aplatie ou parallélépipédique présentant une face transparente bombée à type de loupe grossissante afin de mieux visualiser le canal central et l'introduction du greffon dans l'aiguille d'implantation.

Bien que le dispositif d'aspiration de greffon ait été décrit avec l'emploi d'un support de type cartésien, on peut très bien imaginer employer de la même manière un support de type SCARA (Selective Compliance Assembly Robot Arm) ou un support articulé à cinq ou six axes.

La présente divulgation propose aussi les clauses suivantes :
Clause 1. Dispositif (14, 14') pour chargement de greffons capillaires dans un instrument d'implantation du type Implanteur de Choï, caractérisé en ce qu'il comporte un élément comprenant :
   - un premier orifice (18a) connecté à un canal (17) débouchant dans une chambre (21) d'évacuation de fluides ;
   - un deuxième orifice (22) apte à communiquer avec la chambre (21) d'évacuation de fluides et à définir un chemin avec le canal (17), de sorte à permettre l'introduction de l'aiguille (5) d'un instrument d'implantation (1) dans ledit chemin par le deuxième orifice (22) ;
   - des moyens de connexion (23) de la chambre (21) d'évacuation de fluides à une source de vide (27) ;
   le premier orifice (18a) étant destiné à être mis en communication avec une première tubulure (20) destinée à véhiculer un greffon (12), de sorte que le greffon (12) soit apte à être inséré dans l'aiguille (5) de l'instrument d'implantation (1).
Clause 2. Dispositif selon la clause précédente, dans lequel la chambre (21) d'évacuation de fluides présente des dimensions (d) telles qu'elle soit apte à permettre à la fois un passage de fluide, tel que l'air ou l'eau, entre le canal (17) et les moyens de connexion (23) de la chambre (21) d'évacuation de fluides à une source de vide (27), et le chargement d'un greffon (12) dans l'aiguille (5) d'un instrument d'implantation (1) du type Implanteur de Choï, introduite dans le canal (17) via le deuxième orifice (22).
Clause 3. Dispositif selon l'une quelconque des clauses précédentes, dans lequel le canal (17) est rectiligne.
Clause 4. Dispositif selon l'une quelconque des clauses précédentes, dans lequel les moyens de connexion (23) de la chambre (21) d'évacuation de fluides à une source de vide (27) sont un conduit (23) de mise sous vide disposé dans le dispositif pour chargement de greffon, sensiblement parallèlement au canal (17), destiné à être connecté à la source de vide par un orifice de sortie (29).
Clause 5. Dispositif selon l'une quelconque des clauses 1 à 3, dans lequel, les moyens de connexion (23) de la chambre (21) d'évacuation de fluides à une source de vide (27) sont un conduit (23) de mise sous vide disposé latéralement et perpendiculairement au canal (17).
Clause 6. Dispositif selon l'une quelconque des clauses précédentes, présentant une forme sensiblement cylindrique avec une face proximale (15), une face distale (16), et une face longitudinale (F) reliant les faces proximale et distale.
Clause 7. Dispositif selon la clause précédente, dans lequel la face proximale (15) comporte le deuxième orifice (22), et la face distale (16) comporte le premier orifice (18a) du canal (17).
Clause 8. Dispositif selon l'une quelconque des clauses précédentes, dans lequel le premier (18a) et le deuxième (22) orifices sont alignés dans l'axe du canal (17).
Clause 9. Dispositif selon l'une quelconque des clauses précédentes, comportant des moyens de connexion (19, 25) à des tubulures (20, 24) permettant de relier le canal (17) et le conduit (23) respectivement à un réservoir (28) de greffons et à une source de vide (27).
Clause 10. Procédé d'utilisation du dispositif selon l'une quelconque des clauses précédentes, comportant les étapes suivantes :
   - on connecte une première tubulure (20) au premier orifice (18a), via un moyen de connexion (19),
   - on introduit l'aiguille (5) d'un instrument d'implantation (1), de manière étanche, dans le deuxième orifice (22), de sorte à ce qu'elle pénètre dans au moins une partie du canal (17),
      on approche l'extrémité libre de la première tubulure (20) au contact d'un greffon (12) disposé en solution dans un réservoir (28), et
   - on met la chambre (21) d'évacuation de fluides sous vide.
Clause 11. Plateau de stockage (34) de greffons configuré pour faciliter l'aspiration d'un greffon (12) dans un dispositif (14, 14') pour le chargement de greffon selon les clauses 1 à 9, le plateau de stockage comportant au moins un puit (35) configuré pour contenir un greffon (12) et dont les dimensions permettent de maintenir le greffon dans une position donnée adaptée pour permettre l'aspiration du greffon dans le dispositif (14, 14') pour le chargement de greffon selon les clauses 1 à 9.
Clause 12. Dispositif d'aspiration (100, 100') de greffons configuré pour insérer un greffon dans un dispositif (14, 14') pour chargement de greffon selon les clauses 1 à 9, le dispositif d'aspiration comportant un support (101) et un logement (102) pour un tube (26) rigide configuré pour être relié à la première tubulure (20) du dispositif (14, 14') pour chargement de greffon selon les clauses 1 à 9, le logement étant mobile par rapport au support et configuré pour être déplacé afin de positionner le tube rigide au contact d'un greffon.
Clause 13. Dispositif d'aspiration selon la clause précédente, comportant : une caméra (107) configurée pour enregistrer une image d'un greffon (12) disposé dans un récipient (28), et un contrôleur (108) configuré pour analyser la position précise du greffon.
Clause 14. Ensemble comprenant un dispositif (14, 14') pour chargement de greffon selon les clauses 1 à 9 et un dispositif d'aspiration (100, 100') de greffon selon les clauses 12 à 13.
Clause 15. Ensemble comprenant un dispositif (14, 14') pour chargement de greffon selon les clauses 1 à 9, un dispositif d'aspiration (100, 100') de greffon selon les clauses 12 à 13 et un plateau de stockage selon la clause 11.

La présente divulgation propose en outre les aspects suivants :
Aspect 1. Plateau de stockage (34) de greffons configuré pour faciliter l'aspiration d'un greffon (12) dans un dispositif (14, 14') pour le chargement de greffon, le plateau de stockage comportant au moins un puit (35) configuré pour contenir un greffon (12) et dont les dimensions permettent de maintenir le greffon dans une position donnée adaptée pour permettre l'aspiration du greffon dans le dispositif (14, 14') pour le chargement de greffon.
Aspect 2. Dispositif d'aspiration (100, 100') de greffons configuré pour insérer un greffon dans un dispositif (14, 14') pour chargement de greffon, le dispositif d'aspiration comportant un support (101) et un logement (102) pour un tube (26) rigide configuré pour être relié à la première tubulure (20) du dispositif (14, 14') pour chargement de greffon, le logement étant mobile par rapport au support et configuré pour être déplacé afin de positionner le tube rigide au contact d'un greffon.
Aspect 3. Dispositif d'aspiration selon l'aspect précédent, comportant :
   - une caméra (107) configurée pour enregistrer une image d'un greffon (12) disposé dans un récipient (28), et
   - un contrôleur (108) configuré pour analyser la position précise du greffon.
Aspect 4. Ensemble comprenant un dispositif (14, 14') pour chargement de greffon et un dispositif d'aspiration (100, 100') de greffon selon les aspects 2 ou 3.
Aspect 5. Ensemble comprenant un dispositif (14, 14') pour chargement de greffon, un dispositif d'aspiration (100, 100') de greffon selon les aspects 2 ou 3 et un plateau de stockage selon l'aspect 1.
Aspect 6. Plateau de stockage selon l'aspect 1 ou dispositif d'aspiration selon l'aspect 2 ou ensemble selon l'aspect 4, dans lequel le dispositif (14, 14') pour chargement de greffons a capillaires dans un instrument d'implantation du type Implanteur de Choï, caractérisé en ce qu'il comporte un élément comprenant :
   - un premier orifice (18a) connecté à un canal (17) débouchant dans une chambre (21) d'évacuation de fluides ;
   - un deuxième orifice (22) apte à communiquer avec la chambre (21) d'évacuation de fluides et à définir un chemin avec le canal (17), de sorte à permettre l'introduction de l'aiguille (5) d'un instrument d'implantation (1) dans ledit chemin par le deuxième orifice (22) ;
   - des moyens de connexion (23) de la chambre (21) d'évacuation de fluides à une source de vide (27) ;
   le premier orifice (18a) étant destiné à être mis en communication avec une première tubulure (20) destinée à véhiculer un greffon (12), de sorte que le greffon (12) soit apte à être inséré dans l'aiguille (5) de l'instrument d'implantation (1).
Aspect 7. Plateau de stockage ou dispositif d'aspiration ou ensemble selon l'aspect 6, dans lequel la chambre (21) d'évacuation de fluides présente des dimensions (d) telles qu'elle soit apte à permettre à la fois un passage de fluide, tel que l'air ou l'eau, entre le canal (17) et les moyens de connexion (23) de la chambre (21) d'évacuation de fluides à une source de vide (27), et le chargement d'un greffon (12) dans l'aiguille (5) d'un instrument d'implantation (1) du type Implanteur de Choï, introduite dans le canal (17) via le deuxième orifice (22).
Aspect 8. Plateau de stockage ou dispositif d'aspiration ou ensemble selon l'aspect 6 ou 7, dans lequel le canal (17) est rectiligne.
Aspect 9. Plateau de stockage ou dispositif d'aspiration ou ensemble selon l'aspect 6, 7 ou 8, dans lequel les moyens de connexion (23) de la chambre (21) d'évacuation de fluides à une source de vide (27) sont un conduit (23) de mise sous vide disposé dans le dispositif pour chargement de greffon, sensiblement parallèlement au canal (17), destiné à être connecté à la source de vide par un orifice de sortie (29).
Aspect 10. Plateau de stockage ou dispositif d'aspiration ou ensemble selon l'aspect 6, 7 ou 8, dans lequel les moyens de connexion (23) de la chambre (21) d'évacuation de fluides à une source de vide (27) sont un conduit (23) de mise sous vide disposé latéralement et perpendiculairement au canal (17).
Aspect 11. Plateau de stockage ou dispositif d'aspiration ou ensemble selon les aspects 6 à 10, présentant une forme sensiblement cylindrique avec une face proximale (15), une face distale (16), et une face longitudinale (F) reliant les faces proximale et distale.
Aspect 12. Plateau de stockage ou dispositif d'aspiration ou ensemble selon les aspects 6 à 11, dans lequel la face proximale (15) comporte le deuxième orifice (22), et la face distale (16) comporte le premier orifice (18a) du canal (17).
Aspect 13. Plateau de stockage ou dispositif d'aspiration ou ensemble selon les aspects 6 à 12, dans lequel le premier (18a) et le deuxième (22) orifices sont alignés dans l'axe du canal (17).
Aspect 14. Plateau de stockage ou dispositif d'aspiration ou ensemble selon les aspects 6 à 13, comportant des moyens de connexion (19, 25) à des tubulures (20, 24) permettant de relier le canal (17) et le conduit (23) respectivement à un réservoir (28) de greffons et à une source de vide (27).

## Revendications

1. Système comprenant :
un dispositif (14, 14') de chargement comprenant :
un canal central (17) s'étendant sur la majeure partie de la longueur du dispositif (14, 14') de chargement ;
un premier orifice (18a) situé sur un premier côté extérieur du dispositif (14, 14') de chargement, le premier orifice (18a) débouchant dans une première extrémité du canal central (17) ;
une chambre (21) d'évacuation de fluide reliée de manière fluidique à une seconde extrémité du canal central (17), la chambre (21) d'évacuation de fluide s'étendant à partir de la seconde extrémité du canal central (17) ;
un deuxième orifice (22) située sur un deuxième côté extérieur du dispositif (14, 14') de chargement, à l'opposé du premier côté extérieur, le deuxième orifice (22) débouchant dans la chambre (21) d'évacuation de fluide ; et
un passage de vide s'étendant depuis la chambre (21) d'évacuation de fluide jusqu'à un troisième orifice (29) situé sur le premier côté extérieur du dispositif (14, 14') de chargement, le passage de vide s'étendant sur la majeure partie de la longueur du dispositif (14, 14') de chargement.

2. Système selon la revendication 1, comprenant en outre une source (28) d'au moins un greffon capillaire (12), la source (28) étant en communication fluidique avec le premier orifice (18a).

3. Système selon la revendication 2, dans lequel la source (28) comprend un robot en communication fluidique avec le premier orifice (18a).

4. Système selon l'une quelconque des revendications 2 à 3, dans lequel la source (28) comprend un plateau (34) en communication fluidique avec le premier orifice (18a).

5. Système selon l'une quelconque des revendications 2 à 4, dans lequel la source (28) comprend un réservoir en communication fluidique avec le premier orifice (18a).

6. Système selon l'une quelconque des revendications précédentes, dans lequel le passage de vide comprend un conduit (23) de vide positionné dans le dispositif (14, 14') de chargement et s'étendant sensiblement parallèlement au canal central (17).

7. Système selon l'une quelconque des revendications précédentes, dans lequel le premier orifice (18a) et le deuxième orifice (22) sont alignés le long d'un axe s'étendant à travers le canal central (17).

8. Système selon l'une quelconque des revendications précédentes, dans lequel le canal central (17), le premier orifice (18a), le deuxième orifice (22), la chambre (21) d'évacuation de fluide et le passage de vide sont en communication fluidique les uns avec les autres.

9. Système selon l'une quelconque des revendications précédentes, comprenant en outre une source de vide (27) en communication fluidique avec le passage de vide.

10. Système selon l'une quelconque des revendications précédentes, comprenant en outre une source de vide (27) configurée pour tirer au moins un greffon capillaire (12) à travers le premier orifice (18a) et dans le canal central (17).

11. Procédé comprenant :
la connexion d'un tube (26) d'un robot à un premier orifice (18a) d'un dispositif (14, 14') de chargement ;
l'insertion d'une aiguille (5) à fente d'un instrument d'implantation (1) à travers un deuxième orifice (22) du dispositif (14, 14') de chargement, de telle sorte que l'aiguille (5) à fente s'étende dans au moins une partie d'un canal central (17) du dispositif (14, 14') de chargement et qu'une fente (11) de l'aiguille (5) à fente soit en communication fluidique avec une chambre (21) d'évacuation de fluide du dispositif (14, 14') de chargement ;
l'application d'une aspiration à travers la chambre (21) d'évacuation de fluide vers la fente (11) de l'aiguille (5) à fente afin de charger au moins un greffon capillaire (12) dans l'aiguille (5) à fente ; et
la séparation de l'instrument d'implantation (1) du dispositif (14, 14') de chargement une fois que ledit au moins un greffon capillaire (12) a été chargé dans l'aiguille (5) à fente.

12. Procédé selon la revendication 11, comprenant en outre la réception dudit au moins un greffon capillaire (12) au niveau du dispositif (14, 14') de chargement depuis le robot via le tube (26).

13. Procédé selon l'une quelconque des revendications 11 à 12, comprenant en outre la réception dudit au moins un greffon capillaire (12) au niveau du dispositif (14, 14') de chargement à partir d'un plateau (34) via le tube (26).

14. Procédé selon l'une quelconque des revendications 11 à 13, comprenant en outre la réception dudit au moins un greffon capillaire (12) au niveau du dispositif (14, 14') de chargement à partir d'un réservoir via le tube (26).

15. Procédé selon l'une quelconque des revendications 11 à 14, comprenant en outre la réception dudit au moins un greffon capillaire (12) et d'un liquide vecteur au niveau du dispositif (14, 14') de chargement via le tube (26).
